(19) Europäisches Patentamt
European Patent Office
Office européen des brevets

(11) Veröffentlichungsnummer: **0 480 258 A2**

(12) # EUROPÄISCHE PATENTANMELDUNG

(21) Anmeldenummer: **91116492.9**

(22) Anmeldetag: **27.09.91**

(51) Int. Cl.[5]: **C07D 213/82**, C07D 213/80, A61K 31/455, A61K 31/44, C07D 401/12

(30) Priorität: **10.10.90 DE 4032147**

(43) Veröffentlichungstag der Anmeldung: **15.04.92 Patentblatt 92/16**

(84) Benannte Vertragsstaaten: **AT BE CH DE DK ES FR GB GR IT LI LU NL SE**

(71) Anmelder: **BAYER AG**

**W-5090 Leverkusen 1 Bayerwerk(DE)**

(72) Erfinder: **Bonse, Gerhard, Dr.**
**Wolfskaul 3**
**W-5000 Köln 80(DE)**
Erfinder: **Jeschke, Peter, Dr.**
**Heymannstrasse 38**
**W-5090 Leverkusen(DE)**
Erfinder: **Lindner, Werner, Dr.**
**Märchenstrasse 39**
**W-5000 Köln 80(DE)**
Erfinder: **Harder, Achim, Dr.**
**Piccoloministrasse 398**
**W-5000 K b ln 80(DE)**
Erfinder: **Mencke, Norbert, Dr.**
**Grunder-Mühle 2**
**W-5090 Leverkusen 3(DE)**

(54) **Verwendung von Substituierten 2-Mercaptonicotinsäurederivaten zur Bekämpfung von Endoparasiten, neue substituierte 2-Mercaptonicotinsäurederivate und Verfahren zu ihrer Herstellung.**

(57) Die vorliegende Erfindung betrifft die Verwendung von Verbindungen der allgemeinen Formel I

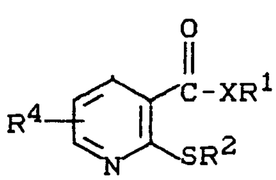

I

in welcher

- $R^1$ für $C_1$-$C_8$ Alkyl-, Aralkyl-, Aryl- oder Heteroarylreste steht die gegebenenfalls durch Halogen, Cyano, Nitro, Amino, Alkylamino, Aminoacyl, Alkyl, Aralkyl, Halogenalkyl, Alkoxy, Aryloxy, Halogenalkoxy, Alkylthio, Arylthio, Alkylsulfinyl, Arylsulfinyl, Arylsulfonyl, Halogenalkylthio, Halogenalkylsulfinyl, Halogenalkylsulfonyl, Carbonylaryl substituiert sind,
- X für O, oder $>$N-$R^3$ steht,
- $R^2$ für $C_{1-5}$-Alkyl, Aralkyl, Alkenyl, Alkinyl steht,
- $R^3$ für H, Alkyl steht oder $R^1$ und $R^3$ gemeinsam mit dem angrenzenden N-Atom für einen 5 oder 6-gliedrigen Heterocyclus stehen, der als weitere Heteroatome 0 oder N enthalten kann und gegebenenfalls durch $C_{1-4}$-Alkyl substituiert ist,
- $R^4$ für Wasserstoff, Halogen, Alkyl, Halogenalkyl, Cyano, Nitro, Amino, Aminoacyl, Phenyl, Alkoxy, Thioalkyl, Aryloxy, Arylthio steht die gegebenenfalls substituiert sind,

zur Bekämpfung von Endoparasiten, neue Verbindungen der Formel I sowie deren Herstellung.

EP 0 480 258 A2

Die vorliegende Erfindung betrifft die Verwendung von substituierten 2-Mercaptonicotinsäurederivaten zur Bekämpfung von Endoparasiten, neue 2-Mercaptonicotinsäurederivate und Verfahren zu ihrer Herstellung.

Substituierte 2-Mercaptonicotinsäurederivate sind bereits bekannt. Es ist jedoch nichts über ihre Verwendung gegen Endoparasiten bekannt (Eur. J. Med. Chem. - Chim. Ther. 20(1985) S. 61-66).

1. Die Verwendung von Verbindungen der allgemeinen Formel I

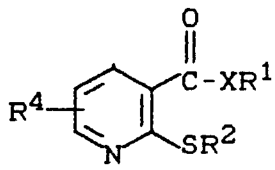

I

in welcher

$R^1$ für $C_1$-$C_8$ Alkyl-, Aralkyl-, Aryl- oder Heteroarylreste steht die gegebenenfalls durch Halogen, Cyano, Nitro, Amino, Aminoacyl, Alkylamino, Alkyl, Aralkyl, Halogenalkyl, Alkoxy, Aryloxy, Halogenalkoxy, Alkylthio, Arylthio, Alkylsulfinyl, Arylsulfinyl, Arylsulfonyl, Halogenalkylthio, Halogenalkylsulfinyl, Halogenalkylsulfonyl, carbonylaryl substituiert sind,

X für O, oder $>$N-$R^3$ steht,

$R^2$ für $C_{1-5}$Alkyl, Aralkyl, Alkenyl, Alkinyl steht,

$R^3$ für H, Alkyl steht oder $R^1$ und $R^3$ gemeinsam mit dem angrenzenden N-Atom für einen 5 oder 6-gliedrigen Heterocyclus stehen, der als weitere Heteroatome 0 oder N enthalten kann und gegebenenfalls durch $C_{1-4}$Alkyl substituiert ist,

$R^4$ für Wasserstoff, Halogen, Alkyl, Halogenalkyl, Cyano, Nitro, Amino, Aminoacyl, Phenyl, Alkoxy, Aryloxy, Thioalkyl, Arylthio steht die gegebenenfalls substituiert sind,

zur Bekämpfung von Endoparasiten in der Medizin und Tiermedizin.

Die Verbindungen der Formel I sind teilweise bekannt und lassen sich analog zu bekannten Verfahren herstellen.

2. Neue substituierte 2-Mercaptonicotinsäurederivate der allgemeinen Formel I

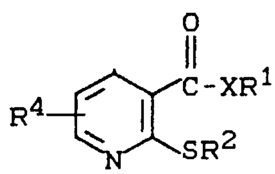

I

in welcher

$R^1$ für Aryl oder Heteroaryl steht die gegebenenfalls durch Halogen, Cyano, Nitro, Amino, Aminoacyl, Alkylamino, Alkyl, Aralkyl, Halogenalkyl, Alkoxy, Aryloxy, Halogenalkoxy, Alkylthio, Arylthio, Alkylsulfinyl, Arylsulfinyl, Arylsulfonyl, Halogenalkylthio, Halogenalkylsulfinyl, Halogenalkylsulfonyl, Carbonylaryl substituiert sind,

X für O, oder den Rest $>$N-$R^3$ steht,

$R^2$ für ($C_2$-$C_5$)Alkyl, Aralkyl, Alkenyl, Alkinyl steht,

$R^3$ für H, Alkyl steht oder $R^1$ und $R^3$ gemeinsam mit dem angrenzenden N-Atom für einen 5 oder 6-gliedrigen Heterocyclus stehen, der als weitere Heteroatome 0 oder N enthalten kann und gegebenenfalls durch $C_{1-4}$Alkyl substituiert ist,

$R^4$ für Wasserstoff, Halogen, Alkyl, Halogenalkyl, Nitro, Amino, Aminoacyl, Cyano, Alkoxy, Aryloxy, Thioalkyl, Arylthio oder Phenyl steht die gegebenenfalls substituiert sind.

3. Verfahren zur Herstellung der Verbindungen der Formel I

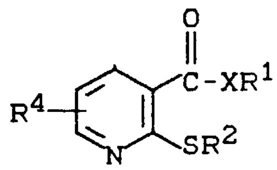

I

in welcher
X, $R^1$, $R^2$, $R^4$ die unter 2 angegebene Bedeutung haben,
dadurch gekennzeichnet, daß man
a) Verbindungen der Formel II

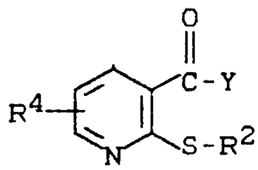

II

in welcher
$R^2$, $R^4$, die oben angegebene Bedeutung besitzen und

Y für Halogen steht,
mit Verbindungen der Formel III

$R^1$-X-H III

in welcher
$R^1$, X die oben angegebenen Bedeutungen besitzen,
umsetzt oder
b) Verbindungen der Formel Ia

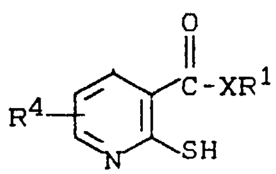

Ia

in welcher
$R^1$, $R^4$ und X die oben angegenen Bedeutungen besitzen,
mit Verbindungen der Formel IV

$R^2$A IV

in welcher
$R^2$ die oben angegebene Bedeutung besitzt jedoch nicht für Wasserstoff steht und

A für Halogen steht,
umsetzt oder
c) Verbindungen der Formel V

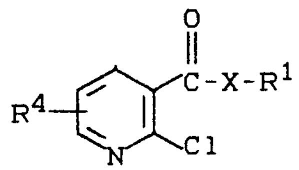

V

in welcher

X, $R^1$, $R^4$, die oben angegebene Bedeutung besitzen

mit Verbindungen der Formel VI

$R^1$-S-H VI

in welcher

$R^1$ die oben angegebene Bedeutung besitzt in Gegenwart einer Base umsetzt.

Die Verbindungen der Formel (I) eignen sich hervorragend zur Bekämpfung von Endoparasiten, besonders auf dem Gebiet der Veterinärmedizin.

Bevorzugt sind Verbindungen der Formel I in welcher

- $R^1$ für $C_{1-4}$-Alkyl, Benzyl, Cycloalkyl mit bis zu 8-C-Atomen, Phenyl, Pyridinyl, Pyrimidinyl, Imidazolyl, Pyrazolyl, Thiophenyl, Furanyl, Thiazolyl, Oxazolyl, Benzthiazolyl, Benzmidazolyl steht, die gegebenenfalls durch einen oder mehrere gleiche oder verschiedene Reste der Gruppe $C_1$-$C_4$-Alkyl, insbesondere Methyl, $C_1$-$C_4$-Alkoxy, insbesondere Methoxy, Ethoxy, Methylendioxy oder Ethylendioxy, die gegebenenfalls durch Fluor oder Chlor substituiert sind, $C_1$-$C_4$-Halogenalkoxy, insbesondere Trifluormethoxy, Fluorchlorethoxy, $C_1$-$C_4$-Halogenalkylthio, insbesondere Trifluormethylthio, Fluorchlormethylthio, $C_1$-$C_4$-Alkylthio, insbesondere Methylthio, Halogensulfonyl, insbesondere Fluorsulfonyl, Thiophenyl, Chlorsulfonyl, $C_1$-$C_4$-Alkylsulfonyl, insbesondere Methylsulfonyl, $C_1$-$C_4$-Halogenalkylsulfonyl, insbesondere Trifluormethylsulfonyl, $C_1$-$C_4$-Halogenalkyl, insbesondere Trifluormethyl, Halogen, insbesondere Fluor oder Chlor, $NO_2$, Cyano, Amino, $C_{1-4}$Alkylamino, $C_{1-4}$-Halogenalkylamino, Acylamino insbesondere Acetylamino, Phenylthio, Phenoxy, die gegebenenfalls durch einen der oben angegebenen Reste substituiert sind,
- X für 0 oder den Rest $>$N-$R^3$ steht,
- $R^2$ für $C_{1-5}$-Alkyl, Benzyl, Ethylphenyl, $C_{2-6}$-Alkenyl steht,
- $R^3$ für $C_{1-5}$-Alkyl steht oder $R^1$ und $R^3$ gemeinsam mit dem angrenzenden N-Atom für einen 5- oder 6-gliedrigen gesättigten Heterocyclus stehen, der als weitere Heteroatome N oder O enthalten kann,
- $R^4$ für Wasserstoff, Halogen, Alkyl oder Phenyl steht das gegebenenfalls durch einen der bei $R^1$ genannten Reste substituiert ist.

Besonders bevorzugt sind Verbindungen der Formel I

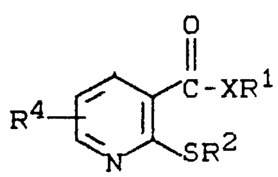

I

in welcher

- $R^1$ die weiter oben angegebene bevorzugte Bedeutung besitzt,
- X für O oder den Rest $>$N-$R^3$ steht,
- $R^2$ für $C_{1-5}$-Alkyl, $C_{2-6}$-Alkenyl steht,
- $R^3$ für Wasserstoff steht,
- $R^4$ für Wasserstoff, Halogen wie Fluor, Chlor, Phenyl, $C_{1-5}$-Alkyl steht.

Ganz besonders bevorzugt sind Verbindungen der Formel I in welcher

- $R^1$ für $C_{1-6}$Alkyl, Cyclopropyl, Cyclopentyl, Cyclohexyl, Benzyl, Phenylethyl, Propylphenyl, Phenyl, Pyridinyl steht die substituiert sind durch Wasserstoff, Halogen, insbesondere Chlor oder Fluor, $C_{1-4}$-Halogenalkyl insbesondere Trifluormethyl, $C_{1-4}$-Halogenalkylthio, insbesondere $SCF_3$, $C_{1-4}$-Alkyl insbesondere Methyl, $C_{1-4}$-Halogenalkoxy insbesondere $OCF_3$, Phenoxy, Carbalkoxy,

Cyano, Nitro, $C_{1-4}$-Alkoxy, $C_{1-4}$-Alkylthio, $C_{1-4}$-Alkylsulfinyl, $C_{1-4}$-Alkylsulfonyl,

X für 0 oder den Rest $>$N-$R^3$ steht,

$R^2$ für $C_{1-5}$-Alkyl insbesondere $C_{1-3}$-Alkyl, $C_{1-6}$-Alkenyl insbesondere Alkyl steht,

$R^3$ für Wasserstoff oder $C_{1-4}$-Alkyl steht,

$R^4$ für Wasserstoff, Chlor, Methyl oder Phenyl steht.

Im einzelnen seien folgende Verbindungen der Formel I genannt in welcher die Reste $R^1$, $R^2$, $R^3$, X die angegebene Bedeutung besitzen:

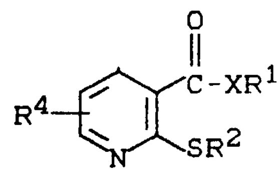

| X | $R^1$ | $R^2$ | $R^4$ |
|---|---|---|---|
| NH | Phenyl | $CH_3$ | 6-$CH_3$ |
| NH | 4-Cl-Phenyl | n-$C_3H_7$ | 6-$CH_3$ |
| NH | Phenyl | n-$C_3H_7$ | 6-$CH_3$ |
| NH | Phenyl | n-$C_3H_7$ | 5-Cl |
| NH | 4-$SCF_3$-Phenyl | $CH_3$ | 6-$CH_3$ |
| NH | 4-$SCF_3$-Phenyl | n-$C_3H_7$ | 5-Cl |
| O | Phenyl | $CH_3$ | H |
| O | Phenyl | n-$C_3H_7$ | H |
| O | Phenyl | n-$C_3H_7$ | 5-Cl |
| O | n-$C_4H_9$ | n-$C_3H_7$ | H |
| NH | 4-Cl-Phenyl | n-$C_3H_7$ | 5-Cl |
| NH | 4-Cl-Phenyl | n-$C_3H_7$ | 6-Phenyl |
| NH | Phenyl | n-$C_3H_7$ | 6-Phenyl |
| NH | -CH($CH_3$)Phenyl | $CH_3$ | 5-Cl |
| NH | -CH($CH_3$)Naphthyl | $CH_3$ | 5-Cl |

Setzt man in Verfahren (3a) als Verbindung der Formel II 2-Thioethyl-nicotinsäurechlorid und als Verbindung der Formel III 4-Trifluormethylanilin ein, so läßt sich das Verfahren durch folgendes Formelschema darstellen.

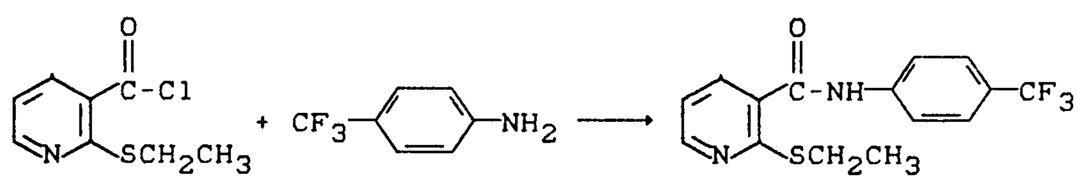

Die Verbindungen der Formel II sind teilweise bekannt. Sie können nach an sich bekannten Verfahren hergestellt werden, z.B. Eur. J. Med. Chem.-Chim. Ther. 20, (1) 61-66 (1985).

Bevorzugt werden Verbindungen der Formel II eingesetzt in denen $R^2$ und $R^4$ die bei den Verbindungen der Formel I angegebenen bevorzugten Bedeutungen besitzen.

Im einzelnen seien folgende Verbindungen der Formel II genannt:

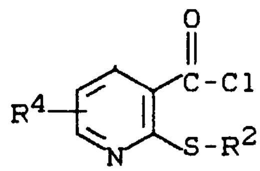

| $R^2$ | $R^4$ |
|---|---|
| $CH_3$ | H |
| $C_2H_5$ | H |
| $C_3H_7$ | H |
| $CH_2$-$CH=CH_2$ | H |
| $CH_3$ | 5-Cl |
| $C_3H_7$ | 5-Cl |
| $CH_3$ | 6-$CH_3$ |
| $CH_3$ | 6-Phenyl |
| $C_3H_7$ | 6-(4-Cl-Phenyl) |
| $C_3H_7$ | 6-(4-$CF_3$-Phenyl) |
| $C_2H_5$ | 6-(4-$CH_3$-Phenyl) |
| $C_3H_7$ | 5-Br |
| $CH_3$ | 5-Br |

Die Reaktion wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und -ethylester, Nitrile wie Essigsäuremethylester und -ethylester, Nitrile wie z.B. Acetonitril und Propionitril, Dimethylacetamid und N-Methyl-pyrrolidon sowie Dimethylsulfoxid, Tetramethylensulfon und Hexamethylphosphorsäuretriamid.

Die Umsetzung erfolgt bei Temperaturen zwischen +20 °C und +180 °C, vorzugsweise zwischen +50 °C und +90 °C.

Das Verfahren wird durchgeführt, indem etwa äquimolare Mengen der Verbindungen der Formel II und III in einem der angegebenen Verdünnungsmittel zusammengegeben und erhitzt werden. Nach vollendeter Umsetzung wird abgekühlt und der ausgefallene Feststoff abfiltriert, gewaschen und getrocknet.

Das erfindungsgemäße Verfahren (3a) wird gegebenenfalls in Gegenwart eines basischen Reaktionshilfsmittels durchgeführt.

Als solche kommen alle üblichen anorganischen oder organischen Basen in Frage, wie beispielsweise Alkalihydroxide, Alkalicarbonate, tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU). Vorzugsweise verwendet man tertiäre Amine, wie beispielsweise Triethylamin.

Setzt man bei Verfahren (3b) als Verbindung der Formel Ia 2-Mercaptonicotinsäure-4-trifluormethylthioanilid und als Verbindung der Formel IV Ethyliodid ein läßt sich das Verfahren durch folgende allgemeine Formel beschreiben

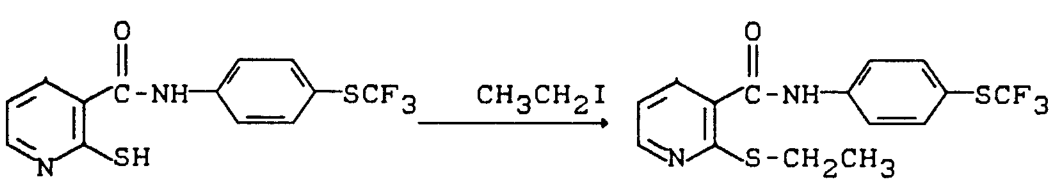


Die Verbindungen der Formel IV sind bekannt, die Herstellung der Verbindung der Formel Ia wird unter (3c) beschrieben.

Bevorzugt werden Verbindungen der Formel Ia eingesetzt in denen $R^1$ und $R^4$ die bei den Verbindun-

gen der Formel I angegebenen besonders bevorzugten Bedeutungen besitzen.

Die Reaktion wird vorzugsweise unter Verwendung von Verdünnungsmitteln durchgeführt.

Als Verdünnungsmittel zur Durchführung des erfindungsgemäßen Verfahrens (3a) kommen vorzugsweise inerte organische Lösungsmittel infrage.

Hierzu gehören insbesondere aliphatische, alicyclische oder aromatische, gegebenenfalls halogenierte Kohlenwasserstoffe, wie beispielsweise Benzin, Ligroin, Benzol, Toluol, Xylol, Chlorbenzol, Petrolether, Pentan, Hexan, Heptan, Cyclohexan, Dichlormethan, Chloroform, Tetrachlorkohlenstoff; Ether, wie Diethylether, Dioxan, Tetrahydrofuran oder Ethylenglykoldimethyl oder -diethylether; Ketone, wie Aceton oder Butanon; Nitrile, wie Acetonitrile oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester oder Basen, wie Pyridin.

Das erfindungsgemäße Verfahren (3b) wird gegebenenfalls in Gegenwart eines basischen Reaktionshilfsmittels durchgeführt.

Als solche kommen alle üblichen anorganischen oder organischen Basen in Frage, wie beispielsweise Alkalihydroxide, Alkalicarbonate, tertiäre Amine, wie Triethylamin, N,N-Dimethylanilin, Pyridin, N,N-Dimethylamino-pyridin, Diazabicyclooctan (DABCO), Diazabicyclononen (DBN) oder Diazabicycloundecen (DBU). Vorzugsweise verwendet man Alkalicarbonate, wie beispielsweise Natriumcarbonat oder Kaliumcarbonat.

Die Reaktionstemperaturen können bei der Durchführung des erfindungsgemäßen Verfahrens (3a) in einem größeren Bereich variiert werden. Im allgemeinen arbeitet man bei Temperaturen zwischen +50 °C und +160 °C, vorzugsweise bei Temperaturen zwischen +60 °C und +90 °C.

Setzt man bei Verfahren (3c) als Verbindung der Formel V 2-Chlor-6-methylnicotinsäuretrifluormethylanilid ein und als Verbindung der Formel VI Propylmercaptan ein läßt sich das Verfahren durch folgendes Formelschema beschreiben

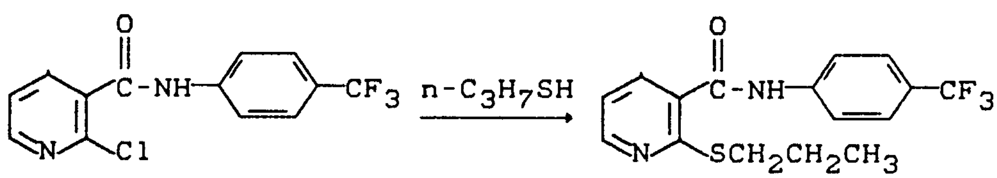


Die Verbindungen der Formel VI sind bekannt; die Verbindungen der Formel V sind bekannt oder können nach bekannten Verfahren hergestellt werden
(Deutsche Offenlegungsschriften DOS 2 611 601, DOS 2 417 216).

Das Verfahren wird durchgeführt, indem etwa äquimolare Mengen der Verbindungen der Formel (V) und (VI) in einem der angegebenen Verdünnungsmittel, gegebenenfalls in Gegenwart einer Base, zusammengegeben und erhitzt werden. Nach vollendeter Umsetzung wird abgekühlt und der ausgefallene Feststoff abfiltriert, gewaschen und getrocknet.

Als Lösungsmittel finden die bei Verfahren (3a) und (3b) vorzugsweise genannten Verwendung. Zusätzlich können Alkohole wie z.B. Ethoxyethanol bevorzugt eingesetzt werden. Als Basen finden die unter Verfahren (3a) und (3b) vorzugsweise Genannten Verwendung. Bevorzugt werden Alkalimetallhydroxide eingesetzt.

Die Wirkstoffe eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella

spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln;

Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß.Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-2}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8/C_{10}$-Fettsäuren.

Fettsäureester wie Ethylstearat, Di-n-butyryl-adipat, Laurinsäurehexylester, Dipropylen-glykolpelargonat,

Ester einer verzweigten Fettsäure mittlerer Kettenlänge mit gesättigten Fettalkoholen der Kettenlänge $C_{16}$-$C_{18}$,Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

Beispiel A

In vivo Nematodentest

Trichostrongylus colubriformis / Schaf

Experimentell mit Trichostrongylus colubriformis infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 2 | 10 |
| 3 | 10 |
| 7 | 5 |
| 13 | 10 |
| 29 | 10 |
| 87 | 10 |

Beispiel B

In vivo Nematodentest

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Würmer vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzeiren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 1 | 10 |
| 2 | 10 |
| 6 | 5 |
| 7 | 10 |
| 9 | 10 |
| 24 | 10 |
| 29 | 10 |

Herstellungsbeispiele

Beispiel für Verfahren 3a

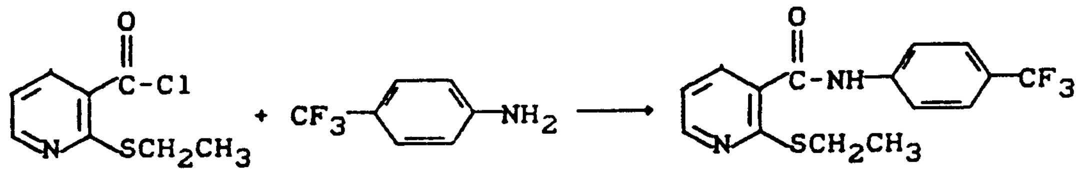

8,7 g (43 mmol) Ethylmercaptonicotinsäurechlorid werden in 120 ml trockenem Tetrahydrofuran gelöst und mit 7,0 g (43 mmol) 4-Trifluormethylanilin und 4,4 g Triethylamin gelöst in 50 ml THF versetzt. Man rührt bei Rückflußtemperatur 3 h, läßt abkühlen, verdünnt mit Wasser und saugt den ausgefallenen Niederschlag ab. Nach der Trocknung resultieren 11,8 g (84%) des Ethylmercaptonicotinsäureanilids.

Beispiel für Verfahren 3b

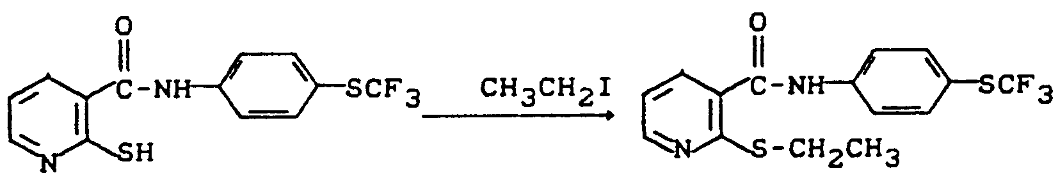


14,3 g (48 mmol) Mercaptonicotinsäureanilid werden in 150 ml trockenem Methanol gelöst und mit 1,92 (48 mmol) NaOH versetzt. Man rührt 10 min bei RT nach und tropft dann 7.1 g (56 mmol) Ethyliodid in 50 ml Methanol zu. Man rührt 1 h bei Raumtemperatur nach versetzt mit Wasser und saugt den ausgefallenen Feststoff ab. Man erhält 12,5 g (73%) der Ethylmercaptoverbindung.

Beispiel für Verfahren 3c

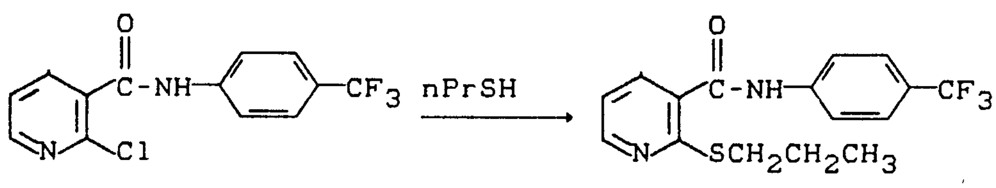


1,9 g (25 mmol) n-Propylmercaptan werden in 100 ml Ethoxyethanol gelöst und mit 1,4 g (25 mmol) KOH versetzt. Zu dieser Lösung tropft man 7,6 g (25 mmol) Chlornicotinsäureanilid und rührt 2 h bei 80 ° C nach. Man kühlt ab und gibt Wasser zu. Ausgefallener Feststoff wird abgesaugt. Man erhält so 6,6 g (77%) Thiopropylnicotinsäureanilid.

Analog zu diesen Verfahren können die folgenden Verbindungen hergestellt werden:

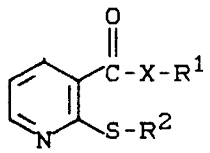

| Bsp. Nr. | X | $R^1$ | $R^2$ | Fp [° C] |
|---|---|---|---|---|
| 1 | NH | Phenyl | $CH_3$ | 164 |
| 2 | NH | Phenyl | $n\text{-}C_3H_7$ | 121 |
| 3 | NH | Phenyl | $CH_2\text{-}CH\text{=}CH_2$ | 137 |
| 4 | NH | 2-Cl-Phenyl | $CH_3$ | 102 |
| 5 | NH | 2-Cl-Phenyl | $CH_2\text{-}CH\text{=}CH_2$ | 91 |
| 6 | NH | 2-Cl-Phenyl | $n\text{-}C_3H_7$ | 98 |
| 7 | NH | 4-Cl-Phenyl | $CH_3$ | 158 |
| 8 | NH | 4-Cl-Phenyl | $CH_2\text{-}CH\text{=}CH_2$ | 138 |
| 9 | NH | 4-Cl-Phenyl | $n\text{-}C_3H_7$ | 138 |
| 10 | NH | 2-$OCF_3$-Phenyl | $CH_3$ | 120 |
| 11 | NH | 2-$OCF_3$-Phenyl | $CH_2\text{-}CH\text{=}CH_2$ | 100 |
| 12 | NH | 3-$CF_3$-Phenyl | $n\text{-}C_3H_7$ | 86 |
| 13 | NH | 4-Cl-Phenyl | $C_2H_5$ | 143 |
| 14 | NH | 2-Cl-Phenyl | $C_2H_5$ | 110 |
| 15 | NH | Phenyl | $C_2H_5$ | 157 |
| 16 | NH | 4-$CF_3$-Phenyl | $C_2H_5$ | 154 |
| 17 | NH | 4-$SCF_3$-Phenyl | $C_2H_5$ | 156 |
| 18 | NH | 2-$OCF_3$-Phenyl | $C_2H_5$ | 103 |

| Bsp. Nr. | X | $R^1$ | $R^2$ | Fp [°C] |
|---|---|---|---|---|
| 19 | NH | 2-$OCF_3$-Phenyl | n-$C_3H_7$ | 90 |
| 20 | NH | 4-$SCF_3$-Phenyl | n-$C_3H_7$ | 121 |
| 21 | NH | Phenyl | n-$C_5H_{11}$ | 91 |
| 22 | NH | 2-Cl-Phenyl | n-$C_5H_{11}$ | 82 |
| 23 | NH | 4-Cl-Phenyl | n-$C_5H_1$ | 130 |
| 24 | NH | Phenyl | $C_2H_5$ | 157 |
| 25 | NH | 4-$CF_3$-Phenyl | $C_2H_5$ | 154 |
| 26 | NH | 4-$SCF_3$-Phenyl | $C_2H_5$ | 156 |
| 27 | NH | 2-$OCF_3$-Phenyl | $C_2H_5$ | 103 |
| 28 | NH | 2-$OCF_3$-Phenyl | n-$C_3H_7$ | 90 |
| 29 | NH | 4-$SCF_3$-Phenyl | n-$C_3H_7$ | 121 |
| 30 | NH | Phenyl | n-$C_5H_{11}$ | 91 |
| 31 | NH | 2-Cl-Phenyl | n-$C_5H_{11}$ | 82 |
| 32 | NH | 4-Cl-Phenyl | n-$C_5H_{11}$ | 130 |
| 33 | NH | 2-$OCF_3$-Phenyl | n-$C_5H_{11}$ | 80 |
| 34 | NH | 4-$SCF_3$-Phenyl | $CH_3$ | 158 |
| 35 | NH | 2-$CH_3$-Phenyl | $CH_2$-CH=$CH_2$ | 114 |
| 36 | NH | 4-$CH_3$-Phenyl | $CH_2$-CH=$CH_2$ | 128 |
| 37 | NH | 2-$CH_3$-Phenyl | $C_2H_5$ | 139 |
| 38 | NH | 2-$CH_3$-Phenyl | n-$C_3H_7$ | 105 |

| Bsp. Nr. | X | $R^1$ | $R^2$ | Fp [°C] |
|---|---|---|---|---|
| 39 | NH | 4-$CH_3$-Phenyl | $C_2H_5$ | 123 |
| 40 | NH | 4-$CH_3$-Phenyl | n-$C_3H_7$ | 127 |
| 41 | NH | Cy-$C_6H_{11}$ | $C_2H_5$ | 128 |
| 42 | NH | Cy-$C_6H_{11}$ | n-$C_5H_{11}$ | 107 |
| 43 | NH | Cy-$C_6H_{11}$ | $CH_3$ | 169 |
| 44 | NH | 3-Cl, 2-$CH_3$-Phenyl | n-$C_3H_7$ | 93 |
| 45 | NH | Cy-$C_6H_{11}$ | n-$C_3H_7$ | 102 |
| 46 | NH | 3-Cl, 2-$CH_3$-Phenyl | $CH_2$-CH=$CH_2$ | 63 |
| 47 | NH | Cy-$C_6H_{11}$ | $CH_2$-CH=$CH_2$ | 122 |
| 48 | NH | 2-$CF_3$-Phenyl | $C_2H_5$ | 93 |
| 49 | NH | 2-$CH_3$-Phenyl | $CH_3$ | 151 |
| 50 | NH | 3-$CF_3$-Phenyl | $CH_2$-CH=$CH_2$ | |
| 51 | NH | 3-$CF_3$-Phenyl | $CH_3$ | 121 |
| 52 | NH | 4-$CH_3$-Phenyl | $CH_3$ | 176 |
| 53 | NH | 2-$CH_3$, Cl-Phenyl | $CH_3$ | 128 |
| 54 | NH | 2-$CH_3$-Phenyl | n-$C_5H_{11}$ | 120 |
| 55 | NH | 4-$CH_3$-Phenyl | n-$C_5H_{11}$ | 74 |
| 56 | NH | 2-Cl, 3-$CH_3$-Phenyl | n-$C_5H_{11}$ | |
| 57 | NH | 3-$CF_3$-Phenyl | n-$C_5H_{11}$ | |
| 58 | NH | i-$C_3H_7$ | $CH_3$ | 138 |
| 59 | NH | i-$C_3H_7$ | $C_2H_5$ | 107 |
| 60 | NH | n-$C_4H_9$ | $C_2H_5$ | 61 |

| Bsp. Nr. | X | $R^1$ | $R^2$ | Fp [°C] |
|---|---|---|---|---|
| 61 | NH | $t-C_4H_9$ | $C_2H_5$ | 141 |
| 62 | NH | $i-C_3H_7$ | $CH_2-CH=CH_2$ | 92 |
| 63 | NH | $n-C_4H_9$ | $CH_2-CH=CH_2$ | 47 |
| 64 | NH | $n-C_5H_{11}$ | $C_2H_5$ | 52 |
| 65 | NH | $i-C_3H_7$ | $n-C_3H_7$ | 82 |
| 66 | NH | $n-C_4H_9$ | $n-C_3H_7$ | 49 |
| 67 | NH | $t-C_4H_9$ | $n-C_3H_7$ | 116 |
| 68 | NH | $n-C_5H_9$ | $n-C_3H_7$ | |
| 69 | NH | $t-C_4H_9$ | $CH_3$ | 131 |
| 70 | NH | $n-C_5H_{11}$ | $CH_3$ | 58 |
| 71 | NH | $t-C_4H_9$ | $CH_2-CH=CH_2$ | 110 |
| 72 | NH | $n-C_5H_{11}$ | $CH_2-CH=CH_2$ | 97 |
| 73 | NH | $n-C_4H_9$ | $CH_3$ | 58 |
| 74 | NH | $i-C_3H_7$ | $C_5H_{11}$ | 62 |
| 75 | NH | $n-C_4H_9$ | $n-C_4H_9$ | |
| 76 | NH | $n-C_5H_{11}$ | $n-C_4H_9$ | |
| 77 | NH | $i-C_3H_7$ | $n-C_5H_{11}$ | 99 |
| 78 | NH | 4-F, 3-Cl-Phenyl | $C_2H_5$ | 153 |
| 79 | NH | 2,5-$CH_3$-Phenyl | $C_2H_5$ | 142 |
| 80 | NH | 2,3-$CH_3$-Phenyl | $n-C_3H_7$ | 126 |
| 81 | NH | 3-Cl, 4-F-Phenyl | $n-C_3H_7$ | 126 |

| Bsp. Nr. | X | $R^1$ | $R^2$ | Fp [°C] |
|---|---|---|---|---|
| 82 | NH | 3-$OCH_3$-Phenyl | $CH_3$ | 129 |
| 83 | NH | 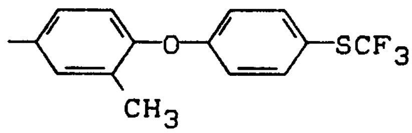 | $CH_3$ | 163 |
| 84 | NH | 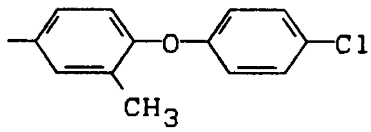 | $CH_3$ | 121 |
| 85 | NH | 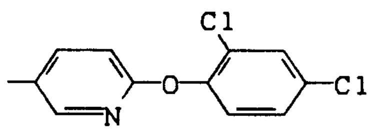 | $CH_3$ | 156 |
| 86 | NH | 3-$CH_3$-Phenyl | $CH_3$ | 125 |
| 87 | NH | 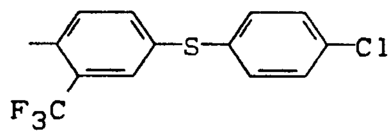 | $CH_3$ | 132 |
| 88 | NH | 3-Cl-Phenyl | $CH_3$ | 98 |
| 89 | NH | 2-$CH_3$-Phenyl | $CH_3$ | 104 |
| 90 | NH | 4-$CH_3$-Phenyl | $CH_3$ | 101 |
| 91 | NH | 2-Cl-Phenyl | -$CH_2$-Phenyl | 126 |
| 92 | NH | 2-$OCH_3$-Phenyl | $CH_3$ | 108 |
| 93 | NH | 4-$OCH_3$-Phenyl | $CH_3$ | 123 |
| 94 | NH | 4-$CF_3$-Phenyl | $CH_3$ | 96 |
| 95 | NH | -$CH_2$-Phenyl | $CH_3$ | 112 |
| 96 | NH | -CH($CH_3$)-Phenyl | $CH_3$ | 109 |

| Bsp. Nr. | X | $R^1$ | $R^2$ | Fp [°C] |
|---|---|---|---|---|
| 97 | NH | $-CH(CH_2)_2$-Phenyl | $CH_3$ | 105 |
| 98 | NH | 3,4-Cl-Phenyl | n-$C_3H_7$ | 98 |
| 99 | NH | 2,4-Cl-Phenyl | n-$C_3H_7$ | 113 |
| 100 | NH | 2-$CH_3$-Phenyl | n-$C_3H_7$ | 108 |
| 101 | NH | 2-Cl-Phenyl | n-$C_3H_7$ | 133 |
| 102 | NH | 2-$CH_3$-Phenyl | n-$C_3H_7$ | 77 |
| 103 | NH | 3-$OCH_3$-Phenyl | n-$C_3H_7$ | 82 |
| 104 | NH | 4-F-Phenyl | n-$C_3H_7$ | 87 |
| 105 | NH | 2-F-Phenyl | n-$C_3H_7$ | 102 |
| 106 | NH | 4-$OCH_3$-Phenyl | n-$C_3H_7$ | 95 |
| 107 | NH | 4-$CH_3$-Phenyl | n-$C_3H_7$ | 103 |
| 108 | NH | 4-$C_2H_5$-Phenyl | n-$C_3H_7$ | 97 |
| 109 | $NCH_3$ | Phenyl | n-$C_3H_7$ | Öl |
| 110 | NH | 3-Cl-Phenyl | n-$C_3H_7$ | 84 |
| 111 | NH | 4-Biphenyl | n-$C_3H_7$ | 134 |
| 112 | NH | 4-Cl-Phenyl | n-$C_3H_7$ | 92 |
| 113 | NH | 1-Naphthyl | n-$C_3H_7$ | 141 |
| 114 | NH | 3,4-Cl-Phenyl | $C_2H_5$ | 105 |
| 115 | NH | 2-$CH_3$-Phenyl | $C_2H_5$ | 118 |
| 116 | NH | 2-Cl-Phenyl | $C_2H_5$ | 126 |
| 117 | NH | 3-$CH_3$-Phenyl | $C_2H_5$ | 96 |

| Bsp. Nr. | X | $R^1$ | $R^2$ | Fp [°C] |
|---|---|---|---|---|
| 118 | NH | 4-F-Phenyl | $C_2H_5$ | 101 |
| 119 | NH | 4-$OCH_3$-Phenyl | $C_2H_5$ | 102 |
| 120 | NH | 4-$CH_3$-Phenyl | $C_2H_5$ | 97 |
| 121 | NH | 3-Cl-Phenyl | $C_2H_5$ | 95 |
| 122 | NH | -CH($CH_3$)-(4-Biphenyl) | $C_2H_5$ | 139 |
| 123 | NH | -CH($CH_3$)-(4-Cl-Phenyl] | $C_2H_5$ | 94 |
| 124 | NH | -CH($CH_3$)-(4-$C_2H_5$-Phenyl) | $C_2H_5$ | 103 |
| 125 | NH | 4-$NHCOCH_3$-Phenyl | $CH_3$ | 255 |
| 126 | NH | 4-$NHCOCH_3$-Phenyl | n-$C_3H_7$ | 212 |
| 127 | NH | 4-$SO_2CH_3$-Phenyl | n-$C_3H_7$ | 165 |
| 128 | NH | 4-$SCH_3$-Phenyl | n-$C_3H_7$ | 126 |
| 129 | NH | 4-$OCH_3$-Phenyl | n-$C_3H_7$ | 112 |
| 130 | NH | 4-$CO_2CH_3$-Phenyl | n-$C_3H_7$ | 119 |
| 131 | NH | 4-F-Phenyl | $CH_3$ | 165 |
| 132 | NH | 4-$CO_2CH_3$-Phenyl | $CH_3$ | 118 |
| 132 | NH | 4-$CO_2CH_3$-Phenyl | $CH_3$ | 118 |
| 133 | NH | 4-CN-Phenyl | n-$C_3H_7$ | 103 |
| 134 | NH | 4-$NO_2$-Phenyl | n-$C_3H_7$ | 115 |
| 135 | NH | 4-CN-Phenyl | $CH_3$ | 179 |
| 136 | NH | 4-$NO_2$-Phenyl | $CH_3$ | 143 |
| 137 | NH | 4-$SO_2CH_3$-Phenyl | $CH_3$ | 121 |
| 138 | NH | 4-$SCH_3$-Phenyl | $CH_3$ | 175 |

| Bsp. Nr. | X | $R^1$ | $R^2$ | Fp [°C] |
|---|---|---|---|---|
| 139 | NH | 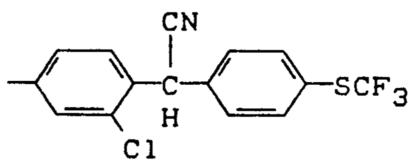 | $CH_3$ | 175 |
| 140 | NH | 2-$CH_3$, 3-Cl-Phenyl | $C_2H_5$ | 92 |
| 141 | NH | -$CH_2$-(2-Cl-Phenyl) | $C_2H_5$ | 101 |
| 142 | NH | -$CH_2$-(2-Cl-Phenyl) | n-$C_3H_7$ | 99 |
| 143 | NH | -$CH_2$-(2-Cl-Phenyl) | n-$C_5H_{11}$ | 96 |
| 144 | NH | -$CH_2$-(2-Cl-Phenyl) | $CH_3$ | 112 |
| 145 | NH | -$CH_2$-(2-Cl-Phenyl) | $CH_2$-CH=$CH_2$ | 74 |
| 146 | NH | -$CH_2$-CH($C_2H_5$)-n-$C_4H_9$ | $C_2H_5$ | Öl |
| 147 | NH | -$CH_2$-CH($C_2H_5$)-n-$C_4H_9$ | n-$C_3H_7$ | Öl |
| 148 | NH | 4-$SCF_3$-Phenyl | $CH_2$-CH=$CH_2$ | 92 |
| 149 | NH | 4-$SCF_3$-Phenyl | n-$C_5H_{11}$ | 88 |
| 150 | NH | -$CH_2$-Phenyl | n-$C_3H_7$ | 116 |
| 151 | NH | 2,4,6-$Cl_3$-Phenyl | n-$C_3H_7$ | 62 |
| 152 | NH | 4-$OCH_3$-Phenyl | n-$C_3H_7$ | 127 |
| 153 | NH | 2,6-$Cl_2$-Phenyl | n-$C_3H_7$ | Öl |
| 154 | NH | -$CH_2$-Phenyl | $CH_3$ | 122 |
| 155 | NH | -$CH_2$-Phenyl | n-$C_5H_{11}$ | 91 |
| 156 | NH | -$CH_2$-Phenyl | $C_2H_5$ | 122 |
| 157 | NH | 4-F-Phenyl | n-$C_3H_7$ | 112 |
| 158 | NH | 4-$OCH_3$-Phenyl | $C_2H_5$ | 135 |

| Bsp. Nr. | X | $R^1$ | $R^2$ | Fp [°C] |
|---|---|---|---|---|
| 159 | NH | 2,4,6-$Cl_3$-Phenyl | $CH_3$ | 182 |
| 160 | NH | 2,4,6-$Cl_3$-Phenyl | n-$C_5H_{11}$ | 64 |
| 161 | NH | -$CH_2$-Phenyl | $CH_2$-CH=$CH_2$ | 117 |
| 162 | NH | 2,6-$Cl_2$-Phenyl | n-$C_5H_{11}$ | Öl |
| 163 | NH | 4-F-Phenyl | $C_2H_5$ | 99 |
| 164 | NH | 4-Cl, 2-F-Phenyl | n-$C_3H_7$ | 96 |
| 165 | NH | 3-Cl, 2-$NO_2$-Phenyl | n-$C_3H_7$ | 109 |
| 166 | NH | 2,4-$F_2$-Phenyl | n-$C_3H_7$ | 110 |
| 167 | NH | -CH($CH_3$)-Phenyl | n-$C_3H_7$ | 109 |
| 168 | NH | 4-$OCH_3$-Phenyl | n-$C_5H_{11}$ | 111 |
| 169 | NH | 4-F-Phenyl | $CH_3$ | 168 |
| 170 | NH | 4-F-Phenyl | $CH_2$-CH=$CH_2$ | 82 |
| 171 | NH | 2,4-$F_2$-Phenyl | $CH_2$-CH=$CH_2$ | 96 |
| 172 | NH | 4-F-Phenyl | n-$C_5H_{11}$ | 112 |
| 173 | NH | 2-Cl, 4-F-Phenyl | $C_2H_5$ | 114 |
| 174 | NH | 3-Cl, 2-$NO_2$-Phenyl | $C_2H_5$ | 112 |
| 175 | NH | 2,4-$F_2$-Phenyl | $C_2H_5$ | 113 |
| 176 | NH | -CH($CH_3$)-Phenyl | $C_2H_5$ | 98 |
| 177 | NH | 4-Cl, 2-F-Phenyl | $CH_3$ | 130 |
| 178 | NH | 3-Cl, 2-$NO_2$-Phenyl | $CH_3$ | 211 |
| 179 | NH | 2,4-$F_2$-Phenyl | $CH_3$ | 105 |

| Bsp. Nr. | X | $R^1$ | $R^2$ | Fp [°C] |
|---|---|---|---|---|
| 180 | NH | Pyrid-2-yl | $CH_2-CH=CH_2$ | 96 |
| 181 | NH | 2-F, 4-Cl-Phenyl | $CH_2-CH=CH_2$ | 82 |
| 182 | NH | Pyrid-2-yl | $n-C_5H_{11}$ | Öl |
| 183 | NH | $-CH(CH_3)-Phenyl$ | $CH_2-CH=CH_2$ | 106 |
| 184 | NH | $2,4-F_2-Phenyl$ | $n-C_5H_{11}$ | 87 |
| 185 | NH | $-CH(CH_3)-Phenyl$ | $n-C_5H_{11}$ | 106 |
| 186 | NH | $-(CH_2)_3-\underset{H}{N}-$ 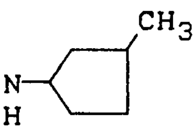 | $C_2H_5$ | Öl |
| 187 | NH | $-(CH_2)_3-\underset{H}{N}-$ 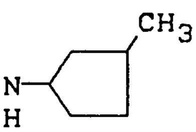 | $n-C_3H_7$ | Öl |
| 188 | NH | $-(CH_2)_2-\underset{H}{N}-(3,4-Cl_2-Phenyl)$ | $C_2H_5$ | 117 |
| 189 | NH | $-(CH_2)_2-\underset{H}{N}-(3,4-Cl_2-Phenyl)$ | $n-C_3H_7$ | 110 |
| 190 | NH | 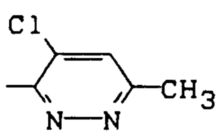 | $C_2H_5$ | 87 |
| 191 | NH | $-(CH_2)_2-N(CH_3)-Phenyl$ | $C_2H_5$ | 75 |
| 192 | NH | $-(CH_2)_2-N(CH_3)-Phenyl$ | $n-C_3H_7$ | 69 |
| 193 | O | $C_2H_5$ | $n-C_3H_7$ | 67 |
| 194 | O | $C_2H_5$ | $C_2H_5$ | 62 |

| Bsp. Nr. | X | $R^1$ | $R^2$ | Fp [° C] |
|---|---|---|---|---|
| 195 | O | $CH_3$ | $n-C_3H_7$ | 115 |
| 196 | O | $CH_3$ | $CH_3$ | 58 |
| 197 | O | $CH_3$ | $CH_2-CH=CH_2$ | Öl |
| 198 | O | $CH_3$ | $n-C_5H_{11}$ | Öl |
| 199 | O | $C_2H_5$ | $n-C_5H_{11}$ | Öl |
| 200 | O | $C_2H_5$ | $CH_3$ | 44 |
| 201 | O | $C_2H_5$ | $CH_2-CH=CH_2$ | |
| 202 | O | Phenyl | $CH_3$ | 86 |
| 203 | O | Phenyl | $CH_2-CH=CH_2$ | |
| 204 | O | Phenyl | $n-C_3H_7$ | 58 |
| 205 | O | Phenyl | $n-C_5H_{11}$ | 41 |
| 206 | O | 4-$CH_3$-Phenyl | $CH_3$ | 101 |
| 207 | O | 4-$CH_3$-Phenyl | $CH_2-CH=CH_2$ | 56 |
| 208 | O | 4-$CH_3$-Phenyl | $n-C_3H_7$ | 32 |
| 209 | O | 4-$CH_3$-Phenyl | $n-C_5H_{11}$ | Öl |
| 210 | O | 4-$OCH_3$-Phenyl | $n-C_3H_7$ | 73 |
| 211 | O | 4-$OCH_3$-Phenyl | $CH_3$ | 100 |
| 212 | O | 4-$OCH_3$-Phenyl | $CH_2-CH=CH_2$ | 85 |
| 213 | O | 4-Cl-Phenyl | $n-C_5H_{11}$ | Öl |
| 214 | O | 4-Cl-Phenyl | $CH_2-CH=CH_3$ | 42 |
| 215 | O | 4-Cl-Phenyl | $C_2H_5$ | 69 |

| Bsp. Nr. | X | $R^1$ | $R^2$ | Fp [°C] |
|---|---|---|---|---|
| 216 | O | 2-$CH_3$-Phenyl | $C_2H_5$ | Öl |
| 217 | O | 4-$CH_3$-Phenyl | $C_2H_5$ | 66 |
| 218 | O | i-$C_3H_7$ | n-$C_3H_7$ | 42 |
| 219 | O | 4-$OCH_3$-Phenyl | $C_2H_5$ | 42 |

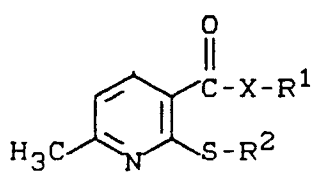

| | | | | |
|---|---|---|---|---|
| 220 | NH | 4-$CH_3$-Phenyl | $CH_3$ | 159 |
| 221 | NH | 4-$CH_3$-Phenyl | $C_2H_5$ | 155 |
| 222 | NH | 2-$CH_3$-Phenyl | $CH_3$ | 145 |
| 223 | NH | 2-$CH_3$-Phenyl | $C_2H_5$ | 111 |
| 224 | NH | 2-$CH_3$-Phenyl | i-$C_3H_7$ | 116 |
| 225 | NH | 2-$CH_3$-Phenyl | n-$C_3H_7$ | 99 |
| 226 | NH | 4-$CH_3$-Phenyl | n-$C_3H_7$ | 129 |
| 227 | NH | 4-$CH_3$-Phenyl | i-$C_3H_7$ | 113 |
| 228 | NH | Phenyl | $CH_3$ | 159 |
| 229 | NH | Phenyl | $C_2H_5$ | 135 |
| 230 | NH | Phenyl | n-$C_3H_7$ | 153 |
| 231 | NH | Phenyl | i-$C_3H_7$ | 119 |
| 232 | NH | 2-$OCH_3$-Phenyl | $CH_3$ | 94 |

| Bsp. Nr. | X | $R^1$ | $R^2$ | Fp [°C] |
|---|---|---|---|---|
| 233 | NH | 2-$OCH_3$-Phenyl | $C_2H_5$ | Öl |
| 234 | NH | 2-$OCH_3$-Phenyl | n-$C_3H_7$ | Öl |
| 235 | NH | 2-$OCH_3$-Phenyl | i-$C_3H_7$ | Öl |
| 236 | NH | 2,3-$(CH_3)_2$-Phenyl | $CH_3$ | 153 |
| 237 | NH | 2,3-$(CH_3)_2$-Phenyl | $C_2H_5$ | 146 |
| 238 | NH | 2,3-$(CH_3)_2$-Phenyl | n-$C_3H_7$ | 130 |
| 239 | NH | 2,3-$(CH_3)_2$-Phenyl | i-$C_3H_7$ | 126 |
| 240 | NH | 3,4-$(CH_3)_2$-Phenyl | $CH_3$ | 147 |
| 241 | NH | 3,4-$(CH_3)_2$-Phenyl | $C_2H_5$ | 127 |
| 242 | NH | 3,4-$(CH_3)_2$-Phenyl | n-$C_3H_7$ | 112 |
| 243 | NH | 3,4-$(CH_3)_2$-Phenyl | i-$C_3H_7$ | 87 |
| 244 | NH | 4-$OCH_3$-Phenyl | $CH_3$ | 156 |
| 245 | NH | 4-$OCH_3$-Phenyl | $C_2H_5$ | 143 |
| 246 | NH | 4-$OCH_3$-Phenyl | n-$C_3H_7$ | 145 |
| 247 | NH | 4-$OCH_3$-Phenyl | i-$C_3H_7$ | 147 |
| 248 | NH | 2-Cl-Phenyl | $CH_3$ | 129 |
| 249 | NH | 2-Cl-Phenyl | $C_2H_5$ | 106 |
| 250 | NH | 2-Cl-Phenyl | n-$C_3H_7$ | 87 |
| 251 | NH | 2-Cl-Phenyl | i-$C_3H_7$ | 72 |
| 252 | NH | 3-Cl-Phenyl | $CH_3$ | 157 |

| Bsp. Nr. | X | $R^1$ | $R^2$ | Fp [°C] |
|---|---|---|---|---|
| 253 | NH | 3-Cl-Phenyl | $C_2H_5$ | 123 |
| 254 | NH | 3-Cl-Phenyl | n-$C_3H_7$ | 96 |
| 255 | NH | 3-Cl-Phenyl | i-$C_3H_7$ | Öl |
| 256 | NH | 4-$OCF_3$-Phenyl | $CH_3$ | 162 |
| 257 | NH | 4-$OCF_3$-Phenyl | $C_2H_5$ | 143 |
| 258 | NH | 4-$OCF_3$-Phenyl | n-$C_3H_7$ | 116 |
| 259 | NH | 4-$OCF_3$-Phenyl | i-$C_3H_7$ | 98 |
| 260 | NH | 4-$SCF_3$-Phenyl | $CH_3$ | 168 |
| 261 | NH | 4-$SCF_3$-Phenyl | $C_2H_5$ | 152 |
| 262 | NH | 4-$SCF_3$-Phenyl | n-$C_3H_7$ | 130 |
| 263 | NH | 4-$SCF_3$-Phenyl | i-$C_3H_7$ | 123 |
| 264 | NH | 3,4-$Cl_2$-Phenyl | $CH_3$ | 159 |
| 265 | NH | 3,4-$Cl_2$-Phenyl | $C_2H_5$ | 100 |
| 266 | NH | 3,4-$Cl_2$-Phenyl | n-$C_3H_7$ | 103 |
| 267 | NH | 3,4-$Cl_2$-Phenyl | i-$C_3H_7$ | 108 |
| 268 | NH | 4-$CF_3$-Phenyl | $CH_3$ | 151 |
| 269 | NH | 4-$CF_3$-Phenyl | $C_2H_5$ | 154 |
| 270 | NH | 4-$CF_3$-Phenyl | n-$C_3H_7$ | 154 |
| 271 | NH | 4-$CF_3$-Phenyl | i-$C_3H_7$ | 129 |
| 272 | NH | 4-Cl-Phenyl | $CH_3$ | 142 |
| 273 | NH | 4-Cl-Phenyl | $C_2H_5$ | 128 |

| Bsp. Nr. | X | $R^1$ | $R^2$ | Fp [°C] |
|---|---|---|---|---|
| 274 | NH | 4-Cl-Phenyl | $n-C_3H_7$ | 117 |
| 275 | NH | 2-Cl, 6-$CH_3$-Phenyl | $n-C_3H_7$ | 99 |
| 276 | NH | 3,5-$(CF_3)_2$-Phenyl | $CH_3$ | 145 |
| 277 | NH | 3,5-$(CF_3)_2$-Phenyl | $n-C_3H_7$ | 151 |
| 278 | NH | $-CH_2$-Phenyl | $n-C_3H_7$ | 93 |
| 279 | NH | -CH-Phenyl<br>$CH_3$ | $n-C_3H_7$ | 81 |
| 280 | NH | -CH-Phenyl<br>$CH_3$ | $n-C_3H_7$ | 81 |
| 281 | NH | $-CH_2-CH_2$-Phenyl | $n-C_3H_7$ | 79 |
| 282 | NH | $n-C_3H_7$ | $n-C_3H_7$ | 63 |
| 283 | NH | $i-C_3H_7$ | $n-C_3H_7$ | 85 |
| 284 | NH | $i-C_4H_9$ | $n-C_3H_7$ | 73 |
| 285 | NH | $t-C_4H_9$ | $n-C_3H_7$ | 55 |
| 286 | NH | $Cy-C_6H_{11}$ | $n-C_3H_7$ | 123 |
| 287 | NH | $Cy-C_5H_9$ | $n-C_3H_7$ | 85 |
| 288 | NH | $Cy-C_3H_5$ | $n-C_3H_7$ | 122 |
| 289 | NH | $-CH-Cy-C_6H_{11}$<br>$CH_3$ | $n-C_3H_7$ | 116 |
| 290 | NH | $-C-Cy-C_6H_{11}$<br>$CH_3$ | $n-C_3H_7$ | 116 |
| 291 | NH | 2-$CH_3$, 3-F-Phenyl | $n-C_3H_7$ | 110 |
| 292 | NH | 2-$CH_3$, 3-F-Phenyl | $CH_3$ | 164 |

| Bsp. Nr. | X | $R^1$ | $R^2$ | Fp [°C] |
|---|---|---|---|---|
| 293 | NH | 2-$CH_3$, 3-Cl-Phenyl | n-$C_3H_7$ | 109 |
| 294 | NH | 2,3-$Cl_2$-Phenyl | n-$C_3H_7$ | 103 |

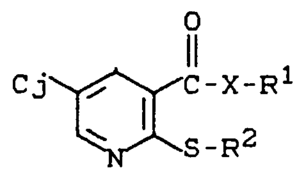

| | | | | |
|---|---|---|---|---|
| 295 | NH | 4-Cl-Phenyl | $CH_3$ | 205 |
| 296 | NH | 4-$CH_3$-Phenyl | $CH_3$ | 173 |
| 297 | NH | 4-$CH_3$-Phenyl | $C_2H_5$ | 188 |
| 298 | NH | 4-$CH_3$-Phenyl | i-$C_3H_7$ | 145 |
| 299 | NH | 4-$CH_3$-Phenyl | n-$C_4H_9$ | 147 |
| 300 | NH | 3-$OCH_3$-Phenyl | $CH_3$ | 154 |
| 301 | NH | 3-$OCH_3$-Phenyl | $C_2H_5$ | 129 |
| 302 | NH | 3-$OCH_3$-Phenyl | i-$C_3H_7$ | 103 |
| 303 | NH | 3-$OCH_3$-Phenyl | n-$C_4H_9$ | 125 |
| 304 | NH | 4-Cl-Phenyl | $C_2H_5$ | 190 |
| 305 | NH | 4-Cl-Phenyl | i-$C_3H_7$ | 187 |
| 306 | NH | 4-Cl-Phenyl | n-$C_4H_9$ | 146 |
| 307 | NH | 4-Cl-Phenyl | n-$C_3H_7$ | 166 |
| 308 | NH | 4-Cl-Phenyl | n-$C_3H_7$ | 148 |
| 309 | NH | 3-$OCH_3$-Phenyl | n-$C_3H_7$ | 141 |

| Bsp. Nr. | X | $R^1$ | $R^2$ | Fp [°C] |
|---|---|---|---|---|
| 310 | NH | $i-C_3H_7$ | $CH_3$ | 162 |
| 311 | NH | $i-C_3H_7$ | $C_2H_5$ | 140 |
| 312 | NH | $i-C_3H_7$ | $i-C_3H_7$ | 121 |
| 313 | NH | $i-C_3H_7$ | $n-C_4H_9$ | 140 |
| 314 | NH | $i-C_3H_7$ | $n-C_3H_7$ | 126 |
| 315 | N | $-CH_2-CH-O-CH-CH_2-$<br>$CH_3$ $CH_3$ | $CH_3$ | 108 |
| 316 | N | $-CH_2-CH-O-CH-CH_2-$<br>$CH_3$ $CH_3$ | $C_2H_5$ | 82 |
| 317 | N | $-CH_2-CH-O-CH-CH_2-$<br>$CH_3$ $CH_3$ | $i-C_3H_7$ | |
| 318 | N | $-CH_2-CH-O-CH-CH_2-$<br>$CH_3$ $CH_3$ | $n-C_4H_9$ | 43 |
| 319 | N | $-CH_2-CH-O-CH-CH_2-$<br>$CH_3$ $CH_3$ | $n-C_3H_7$ | Öl |
| 320 | NH | $3,4-Cl_2$-Phenyl | $CH_3$ | 188 |
| 321 | NH | $3,4-Cl_2$-Phenyl | $C_2H_5$ | 161 |
| 322 | NH | $3,4-Cl_2$-Phenyl | $i-C_3H_7$ | 118 |
| 323 | NH | $3,4-Cl_2$-Phenyl | $n-C_4H_9$ | 145 |
| 324 | NH | $3,4-Cl_2$-Phenyl | $n-C_3H_7$ | 135 |
| 325 | NH | $-CH_2$-Phenyl | $CH_3$ | 151 |

| Bsp. Nr. | X | $R^1$ | $R^2$ | Fp [°C] |
|---|---|---|---|---|
| 325 | NH | $-CH_2$-Phenyl | $CH_3$ | 151 |
| 326 | NH | $-CH_2$-Phenyl | $C_2H_5$ | 142 |
| 327 | NH | $-CH_2$-Phenyl | $i-C_3H_7$ | 117 |
| 328 | NH | $-CH_2$-Phenyl | $n-C_4H_9$ | 132 |
| 329 | NH | $-CH_2$-Phenyl | $n-C_3H_7$ | 155 |
| 330 | NH | 4-F-Phenyl | $CH_3$ | 193 |
| 331 | NH | 4-F-Phenyl | $C_2H_5$ | 178 |
| 332 | NH | 4-F-Phenyl | $i-C_3H_7$ | 161 |
| 333 | NH | 4-F-Phenyl | $n-C_4H_9$ | 146 |
| 334 | NH | 4-F-Phenyl | $n-C_3H_7$ | 155 |
| 335 | NH | $4-SCF_3$-Phenyl | $CH_3$ | 171 |
| 336 | NH | $4-SCF_3$-Phenyl | $C_2H_5$ | 161 |
| 337 | NH | $4-SCF_3$-Phenyl | $i-C_3H_7$ | 123 |
| 338 | NH | $4-SCF_3$-Phenyl | $n-C_4H_9$ | 135 |
| 339 | NH | $4-SCF_3$-Phenyl | $n-C_3H_7$ | 134 |
| 340 | N | $-(CH_2)_2-N(CH_3)-(CH_2)_2-$ | $CH_3$ | 107 |
| 341 | N | $-(CH_2)_2-N(CH_3)-(CH_2)_2-$ | $C_2H_5$ | 73 |
| 342 | N | $-(CH_2)_2-N(CH_3)-(CH_2)_2-$ | $i-C_3H_7$ | 87 |

| Bsp. Nr. | X | $R^1$ | $R^2$ | Fp [°C] |
|---|---|---|---|---|
| 343 | N | $-(CH_2)_2-N-(CH_2)_2-$<br>\|<br>$CH_3$ | $n-C_4H_9$ | 73 |
| 344 | N | $-(CH_2)_2-N-(CH_2)_2-$<br>\|<br>$CH_3$ | $n-C_3H_7$ | 87 |
| 345 | NH | Phenyl | $CH_3$ | 188 |
| 346 | NH | Phenyl | $C_2H_5$ | 180 |
| 347 | NH | Phenyl | $i-C_3H_7$ | 124 |
| 348 | NH | Phenyl | $n-C_4H_9$ | 130 |
| 349 | NH | Phenyl | $n-C_3H_7$ | 142 |
| 350 | NH | 2-Cl-Phenyl | $CH_3$ | 174 |
| 351 | NH | 2-Cl-Phenyl | $C_2H_5$ | 154 |
| 352 | NH | 2-Cl-Phenyl | $1-C_3H_7$ | 111 |
| 353 | NH | 2-Cl-Phenyl | $n-C_4H_9$ | 138 |
| 354 | NH | 2-Cl-Phenyl | $n-C_3H_7$ | 125 |
| 355 | NH | 3-Cl-Phenyl | $CH_3$ | 162 |
| 356 | NH | 3-Cl-Phenyl | $C_2H_5$ | 159 |
| 357 | NH | 3-Cl-Phenyl | $1-C_3H_7$ | 99 |
| 358 | NH | 3-Cl-Phenyl | $n-C_4H_9$ | 100 |
| 359 | NH | 3-Cl-Phenyl | $n-C_3H_7$ | 120 |
| 360 | NH | $2-CH_3$-Phenyl | $CH_3$ | 184 |

| Bsp. Nr. | X | $R^1$ | $R^2$ | Fp [°C] |
|---|---|---|---|---|
| 361 | NH | 2-$CH_3$-Phenyl | $C_2H_5$ | 165 |
| 362 | NH | 2-$CH_3$-Phenyl | i-$C_3H_7$ | 139 |
| 363 | NH | 2-$CH_3$-Phenyl | n-$C_4H_9$ | 155 |
| 364 | NH | 2-$CH_3$-Phenyl | n-$C_3H_7$ | 154 |
| 365 | NH | 3-$CH_3$-Phenyl | $CH_3$ | 179 |
| 366 | NH | 3-$CH_3$-Phenyl | $C_2H_5$ | 147 |
| 367 | NH | 3-$CH_3$-Phenyl | i-$C_3H_7$ | 106 |
| 368 | NH | 3-$CH_3$-Phenyl | n-$C_4H_9$ | 118 |
| 369 | NH | 3-$CH_3$-Phenyl | n-$C_3H_7$ | 140 |
| 370 | NH | -CH($CH_3$)-(4-$CH_3$-Phenyl) | $CH_3$ | 154 |
| 371 | NH | -CH($CH_3$)-(4-$CH_3$-Phenyl) | $C_2H_5$ | 144 |
| 372 | NH | -CH($CH_3$)-(4-$CH_3$-Phenyl) | i-$C_3H_7$ | 132 |
| 373 | NH | -CH($CH_3$)-(4-$CH_3$-Phenyl) | n-$C_4H_9$ | 149 |
| 374 | NH | -CH($CH_3$)-(4-$CH_3$-Phenyl) | n-$C_3H_7$ | 133 |
| 375 | NH | $CH_3$ | $CH_3$ | 159 |
| 376 | NH | $CH_3$ | $C_2H_5$ | 146 |
| 377 | NH | $CH_3$ | i-$C_3H_7$ | 81 |
| 378 | NH | $CH_3$ | n-$C_4H_9$ | 100 |
| 379 | NH | $CH_3$ | n-$C_3H_7$ | 119 |
| 380 | N | $-(CH_2)_5-$ | $CH_3$ | 78 |
| 381 | N | $-(CH_2)_5-$ | $C_2H_5$ | 74 |

| Bsp. Nr. | X | $R^1$ | $R^2$ | Fp [°C] |
|---|---|---|---|---|
| 382 | N | $-(CH_2)_5-$ | $i-C_3H_7$ | 68 |
| 383 | N | $-(CH_2)_5-$ | $n-C_4H_9$ | Öl |
| 384 | N | $-(CH_2)_5-$ | $n-C_3H_7$ | Öl |
| 385 | N | $-(CH_2)_2-O-(CH_2)_2-$ | $CH_3$ | 115 |
| 386 | N | $-(CH_2)_2-O-(CH_2)_2-$ | $C_2H_5$ | 92 |
| 387 | N | $-(CH_2)_2-O-(CH_2)_2-$ | $i-C_3H_7$ | 78 |
| 388 | N | $-(CH_2)_2-O-(CH_2)_2-$ | $n-C_4H_9$ | 68 |
| 389 | N | $-(CH_2)_2-O-(CH_2)_2-$ | $n-C_3H_7$ | 52 |
| 390 | NH | $-CH(CH_3)$-Phenyl | $CH_3$ | 172 |
| 391 | NH | $-CH(CH_3)$-Phenyl | $C_2H_5$ | 142 |
| 392 | NH | $-CH(CH_3)$-Phenyl | $i-C_3H_7$ | 131 |
| 393 | NH | $-CH(CH_3)$-Phenyl | $n-C_4H_9$ | 124 |
| 394 | NH | $-CH(CH_3)$-Phenyl | $n-C_3H_7$ | 144 |
| 395 | NH | $-(CH_2)_7-CH_3$ | $CH_3$ | 90 |
| 396 | NH | $-(CH_2)_7-CH_3$ | $C_2H_5$ | 92 |
| 397 | NH | $-(CH_2)_7-CH_3$ | $i-C_3H_7$ | 71 |
| 398 | NH | $-(CH_2)_7-CH_3$ | $n-C_4H_9$ | 83 |
| 399 | NH | $-(CH_2)_7-CH_3$ | $n-C_3H_7$ | 81 |
| 400 | NH | $Cy-C_6H_11$ | $CH_3$ | 195 |
| 401 | NH | $Cy-C_6H_11$ | $C_2H_5$ | 178 |

| Bsp. Nr. | X | $R^1$ | $R^2$ | Fp [°C] |
|---|---|---|---|---|
| 402 | NH | $Cy-C_6H_{11}$ | $i-C_3H_7$ | 160 |
| 403 | NH | $Cy-C_6H_{11}$ | $n-C_4H_9$ | 142 |
| 404 | NH | $Cy-C_6H_{11}$ | $n-C_3H_7$ | 155 |
| 405 | NH | $2-OCH_3$-Phenyl | $CH_3$ | 122 |
| 406 | NH | $2-OCH_3$-Phenyl | $C_2H_5$ | 94 |
| 407 | NH | $2-OCH_3$-Phenyl | $i-C_3H_7$ | 101 |
| 408 | NH | $2-OCH_3$-Phenyl | $n-C_4H_9$ | 97 |
| 409 | NH | $2,3-(CH_3)_2$-Phenyl | $n-C_3H_7$ | 105 |
| 410 | NH | $4-OCH_3$-Phenyl | $CH_3$ | 191 |
| 411 | NH | $4-OCH_3$-Phenyl | $C_2H_5$ | 189 |
| 412 | NH | $4-OCH_3$-Phenyl | $1-C_3H_7$ | 134 |
| 413 | NH | $4-OCH_3$-Phenyl | $n-C_4H_9$ | 145 |
| 414 | NH | $4-OCH_3$-Phenyl | $n-C_3H_7$ | 143 |
| 415 | NH | $4-OCF_3$-Phenyl | $CH_3$ | 171 |
| 416 | NH | $4-OCF_3$-Phenyl | $C_2H_5$ | 160 |
| 417 | NH | $4-OCF_3$-Phenyl | $i-C_3H_7$ | 113 |
| 418 | NH | $4-OCF_3$-Phenyl | $n-C_4H_9$ | 134 |
| 419 | NH | $4-OCF_3$-Phenyl | $n-C_3H_7$ | 160 |
| 420 | NH | $4-CF_3$-Phenyl | $CH_3$ | 185 |
| 421 | NH | $4-CF_3$-Phenyl | $C_2H_5$ | 202 |
| 422 | NH | $4-CF_3$-Phenyl | $i-C_3H_7$ | 162 |
| 423 | NH | $4-CF_3$-Phenyl | $n-C_4H_9$ | 150 |

| Bsp. Nr. | X | $R^1$ | $R^2$ | Fp [°C] |
|---|---|---|---|---|
| 424 | NH | 4-$CF_3$-Phenyl | n-$C_3H_7$ | 177 |
| 425 | NH | 2,4-$Cl_2$-Phenyl | $CH_3$ | 181 |
| 426 | NH | 2,4-$Cl_2$-Phenyl | $C_2H_5$ | 145 |
| 427 | NH | 2,4-$Cl_2$-Phenyl | i-$C_3H_7$ | 112 |
| 428 | NH | 2,4-$Cl_2$-Phenyl | n-$C_4H_9$ | 128 |
| 429 | NH | 2,4-$Cl_2$-Phenyl | n-$C_3H_7$ | 126 |
| 430 | NH | -CH($CH_3$)-(4-Cl-Phenyl) | $CH_3$ | 166 |
| 431 | NH | -CH($CH_3$)-(4-Cl-Phenyl) | $C_2H_5$ | 143 |
| 432 | NH | -CH($CH_3$)-(4-Cl-Phenyl) | i-$C_3H_7$ | 144 |
| 433 | NH | -CH($CH_3$)-(4-Cl-Phenyl) | n-$C_4H_9$ | 134 |
| 434 | NH | -CH($CH_3$)-(4-Cl-Phenyl) | n-$C_3H_7$ | 126 |
| 435 | O | Phenyl | $CH_3$ | 90 |
| 436 | O | Phenyl | $C_2H_5$ | 91 |
| 437 | O | Phenyl | i-$C_3H_7$ | 88 |
| 438 | O | Phenyl | n-$C_4H_9$ | 48 |
| 439 | O | Phenyl | n-$C_3H_7$ | 69 |
| 440 | O | 4-Cl-Phenyl | $CH_3$ | 112 |
| 441 | O | 4-Cl-Phenyl | $C_2H_5$ | 122 |
| 442 | O | 4-Cl-Phenyl | i-$C_3H_7$ | 77 |
| 443 | O | 4-Cl-Phenyl | n-$C_4H_9$ | 61 |
| 444 | O | 4-Cl-Phenyl | n-$C_3H_7$ | 70 |

| Bsp. Nr. | X | $R^1$ | $R^2$ | Fp [°C] |
|---|---|---|---|---|
| 445 | O | 2-Cl-Phenyl | $CH_3$ | 106 |
| 446 | O | 2-Cl-Phenyl | $C_2H_5$ | 121 |
| 447 | O | 2-Cl-Phenyl | i-$C_3H_7$ | 94 |
| 448 | O | 2-Cl-Phenyl | n-$C_4H_9$ | 79 |
| 449 | O | 2-Cl-Phenyl | n-$C_3H_7$ | 98 |
| 450 | O | 3-Cl-Phenyl | $CH_3$ | 132 |
| 451 | O | 3-Cl-Phenyl | $C_2H_5$ | 123 |
| 452 | O | 3-Cl-Phenyl | i-$C_3H_7$ | 66 |
| 453 | O | 3-Cl-Phenyl | n-$C_4H_9$ | 91 |
| 454 | O | 3-Cl-Phenyl | n-$C_3H_7$ | 109 |
| 455 | O | 4-$OCH_3$-Phenyl | $CH_3$ | 95 |
| 456 | O | 4-$OCH_3$-Phenyl | $C_2H_5$ | 83 |
| 457 | O | 4-$OCH_3$-Phenyl | 1-$C_3H_7$ | Öl |
| 458 | O | 4-$OCH_3$-Phenyl | n-$C_4H_9$ | 57 |
| 459 | O | 4-$OCH_3$-Phenyl | n-$C_3H_7$ | 65 |

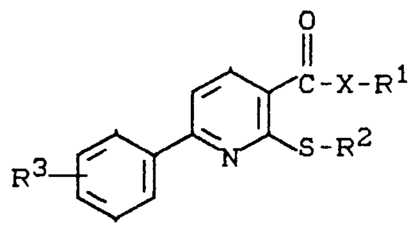

| Bsp. Nr. | X | $R^1$ | $R^2$ | $R^3$ | Fp [°C] |
|---|---|---|---|---|---|
| 460 | NH | 3-Cl-Phenyl | $CH_3$ | 4-Cl | 179 |
| 461 | NH | 3-Cl-Phenyl | n-$C_3H_7$ | H | 139 |
| 462 | NH | Phenyl | $CH_3$ | H | 193 |
| 463 | NH | Phenyl | n-$C_3H_7$ | H | 200 |
| 464 | NH | 4-$CH_3$-Phenyl | $CH_3$ | H | 224 |
| 465 | NH | 4-$CH_3$-Phenyl | n-$C_3H_7$ | H | 204 |
| 466 | NH | 3-Cl-Phenyl | $CH_3$ | H | 143 |
| 467 | NH | 3-Cl-Phenyl | n-$C_3H_7$ | 4-Cl | 167 |
| 468 | NH | 4-$OCH_3$-Phenyl | $CH_3$ | H | 223 |
| 469 | NH | 4-$OCH_3$-Phenyl | n-$C_3H_7$ | H | 201 |
| 470 | NH | -CH($CH_3$)-Phenyl | $CH_3$ | H | 198 |
| 471 | NH | -CH($CH_3$)-Phenyl | n-$C_3H_7$ | H | 198 |
| 472 | NH | 4-Cl-Phenyl | n-$C_3H_7$ | H | 116 |
| 473 | NH | $-(CH_2)_7-CH_3$ | $CH_3$ | H | 124 |
| 474 | NH | $-(CH_2)_7-CH_3$ | n-$C_3H_7$ | H | 114 |
| 475 | NH | 2,4-$Cl_2$-Phenyl | $CH_3$ | H | 190 |

| Bsp. Nr. | X | $R^1$ | $R^2$ | $R^3$ | Fp [°C] |
|---|---|---|---|---|---|
| 476 | N | $-(CH_2)_2-O-(CH_2)_2-$ | $CH_3$ | H | 127 |
| 477 | N | $-(CH_2)_2-O-(CH_2)_2$ | $n-C_3H_7$ | H | 68 |
| 478 | NH | $Cy-C_6H_{11}$ | $CH_3$ | H | 205 |
| 479 | NH | $Cy-C_6H_{11}$ | $n-C_3H_7$ | H | 178 |
| 480 | NH | $2,4-Cl_2$-Phenyl | $n-C_3H_7$ | H | 105 |
| 481 | NH | $CH_3$ | $CH_3$ | H | 191 |
| 482 | NH | $CH_3$ | $n-C_3H_7$ | H | 170 |
| 483 | NH | $4-OCH_3$-Phenyl | $CH_3$ | 4-Cl | 263 |
| 484 | NH | $4-OCH_3$-Phenyl | $n-C_3H_7$ | 4-Cl | 207 |
| 485 | NH | $-CH(CH_3)$-Phenyl | $CH_3$ | 4-Cl | |
| 486 | NH | $-CH(CH_3)$-Phenyl | $n-C_3H_7$ | 4-Cl | 151 |
| 487 | NH | $-(CH_2)_7-CH_3$ | $CH_3$ | 4-Cl | 125 |
| 488 | NH | $-(CH_2)_3-CH_3$ | $n-C_3H_7$ | 4-Cl | 116 |
| 489 | NH | $2,4-Cl_2$-Phenyl | $CH_3$ | 4-Cl | 232 |
| 490 | NH | $2,4-Cl_2$-Phenyl | $n-C_3H_7$ | 4-Cl | 182 |
| 491 | N | $-(CH_2)_2-O-(CH_2)_2-$ | $CH_3$ | 4-Cl | 131 |
| 492 | N | $-(CH_2)_2-O-(CH_2)_2-$ | $n-C_3H_7$ | 4-Cl | Öl |
| 493 | NH | $Cy-C_6H_{11}$ | $CH_3$ | 4-Cl | 205 |
| 494 | NH | $Cy-C_6H_{11}$ | $n-C_3H_7$ | 4-Cl | 230 |
| 495 | NH | $CH_3$ | $CH_3$ | 4-Cl | 205 |
| 496 | NH | $CH_3$ | $n-C_3H_7$ | 4-Cl | 183 |

| Bsp. Nr. | X | $R^1$ | $R^2$ | $R^3$ | Fp [°C] |
|---|---|---|---|---|---|
| 497 | NH | 3-Cl-Phenyl | $CH_3$ | 4-$CH_3$ | 196 |
| 498 | NH | 3-Cl-Phenyl | n-$C_3H_7$ | 4-$CH_3$ | 162 |
| 499 | NH | 4-$OCH_3$-Phenyl | $CH_3$ | 4-$CH_3$ | 219 |
| 500 | NH | 4-$OCH_3$-Phenyl | n-$C_3H_7$ | 4-$CH_3$ | 191 |
| 501 | NH | -CH($CH_3$)-Phenyl | $CH_3$ | 4-$CH_3$ | 182 |
| 502 | NH | -CH($CH_2$)-Phenyl | n-$C_3H_7$ | 4-$CH_3$ | 149 |
| 503 | NH | n-$C_8H_{17}$enyl | $CH_3$ | 4-$CH_3$ | 119 |
| 504 | NH | n-$C_8H_{17}$enyl | n-$C_3H_7$ | 4-$CH_3$ | 104 |
| 505 | NH | Cy-$C_6H_{11}$ | n-$C_3H_7$ | 4-$CH_3$ | 193 |

## Patentansprüche

**1.** Verwendung von Verbindungen der allgemeinen Formel I

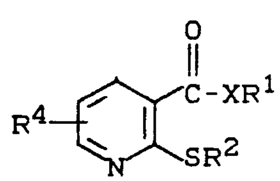

I

in welcher

$R^1$ für $C_1$-$C_8$-Alkyl-, Aralkyl-, Aryl- oder Heteroarylreste steht die gegebenenfalls durch Halogen, Cyano, Nitro, Amino, Alkylamino, Aminoacyl, Alkyl, Aralkyl, Halogenalkyl, Alkoxy, Aryloxy, Halogenalkoxy, Alkylthio, Arylthio, Alkylsulfinyl, Arylsulfinyl, Arylsulfonyl, Halogenalkylthio, Halogenalkylsulfinyl, Halogenalkylsulfonyl, Carbonylaryl

substituiert sind,

X für O, oder $>$N-$R^3$ steht,

$R^2$ für $C_{1-5}$-Alkyl, Aralkyl, Alkenyl, Alkinyl steht,

$R^3$ für H, Alkyl steht oder $R^1$ und $R^3$ gemeinsam mit dem angrenzenden N-Atom für einen 5 oder 6-gliedrigen Heterocyclus stehen, der als weitere Heteroatome 0 oder N enthalten kann und gegebenenfalls durch $C_{1-4}$-Alkyl substituiert ist,

$R^4$ für Wasserstoff, Halogen, Alkyl, Halogenalkyl, Cyano, Nitro, Amino, Aminoacyl, Phenyl, Alkoxy, Aryloxy, Thioalkyl, Arylthio steht die gegebenenfalls substituiert sind,

zur Bekämpfung von Endoparasiten in der Medizin und Tiermedizin.

**2.** Neue substituierte 2-Mercaptonicotinsäurederivate der allgemeinen Formel I

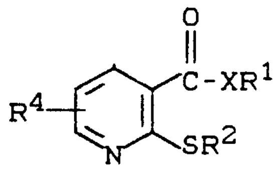

I

in welcher

- $R^1$ für Aryl oder Heteroaryl steht, die gegebenenfalls durch Halogen, Cyano, Nitro, Amino, Alkylamino, Aminoacyl, Alkyl, Aralkyl, Halogenalkyl, Alkoxy, Aryloxy, Halogenalkoxy, Alkylthio, Arylthio, Alkylsulfinyl, Arylsulfinyl, Arylsulfonyl, Halogenalkylthio, Halogenalkylsulfinyl, Halogenalkylsulfonyl, Carbonylaryl substituiert sind.
- X für O, oder den Rest $>N-R^3$ steht,
- $R^2$ für $C_2$-$C_5$-Alkyl, Aralkyl, Alkenyl, Alkinyl steht,
- $R^3$ für H, Alkyl oder $R^1$ und $R^3$ gemeinsam mit dem angrenzenden N-Atom für einen 5 oder 6-gliedrigen Heterocyclus stehen, der als weitere Heteroatome 0 oder N enthalten kann und gegebenenfalls durch $C_{1-4}$-Alkyl substituiert ist,
- $R^4$ für Wasserstoff, Halogen, Alkyl, Halogenalkyl, Nitro, Amino, Aminoacyl, Cyano, Alkoxy, Aryloxy, Thioalkyl, Arylthio oder Phenyl steht die gegebenenfalls substituiert sind.

**3.** Verfahren zur Herstellung der Verbindungen der Formel I

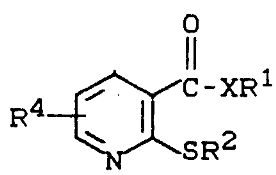

I

in welcher

X, $R^1$, $R^2$, $R^4$ die in Anspruch 2 angegebene Bedeutung haben,

dadurch gekennzeichnet, daß man

a) Verbindungen der Formel II

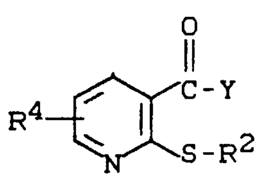

II

in welcher

$R^2$, $R^4$, die oben angegebene Bedeutung besitzen und

Y für Halogen steht,

mit Verbindungen der Formel III

$R^1$-X-H III

in welcher

$R^1$, X die oben angegebenen Bedeutungen besitzen,

umsetzt oder
b) Verbindungen der Formel Ia

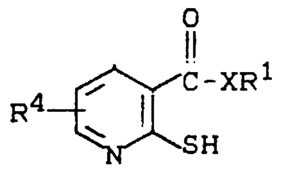

Ia

in welcher

$R^1$, $R^4$ und X die oben angegebenen Bedeutungen besitzen,

mit Verbindungen der Formel IV

$R^2A$ IV

in welcher

$R^2$ die oben angegebene Bedeutung besitzt jedoch nicht für Wasserstoff steht und
A für Halogen steht,
umsetzt oder
c) Verbindungen der Formel V

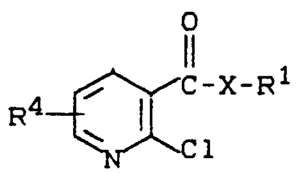

V

in welcher

X, $R^1$, $R^4$, die oben angegebene Bedeutung besitzen

mit Verbindungen der Formel VI

$R^1$-S-H VI

in welcher

$R^1$ die oben angegebene Bedeutung besitzt in Gegenwart einer Base umsetzt.

**4.** Endoparasitizide Mittel, gekennzeichnet durch einen Gehalt an mindestens einem substituierten 2-Mercaptonicotinsäurederivat der Formel (I) gemäß Anspruch 1.

**5.** Verfahren zur Herstellung von endoparasitiziden Mitteln, dadurch gekennzeichnet, daß man substituierte 2-Mercaptonicotinsäurederivate der Formel (I) gemäß Anspruch 1 mit Streckmitteln und/oder oberflächenaktiven Mitteln vermischt.

**6.** Verwendung von substituierten 2-Mercaptonicotinsäurederivaten der Formel (I) gemäß Anspruch 1 zur Herstellung von endoparasitiziden Mitteln.